# FASCICULE DE BREVET EUROPEEN

(11) **EP 2 722 024 B1**
(45) Date de publication et mention de la délivrance du brevet: **06.12.2017**
(21) Numéro de dépôt: 13189062.6
(22) Date de dépôt: 17.10.2013
(51) Int. Cl.: A61F 6/14, A61F 6/18

(54) **DISPOSITIF DE POSE D'UN STÉRILET, AINSI QUE KIT MÉDICAL COMPORTANT UN TEL DISPOSITIF**
GERÄT ZUM EINFÜHREN EINER INTRAUTERINEN VORRICHTUNG, SOWIE MEDIZINISCHES SET, WELCHES DIESES GERÄT ENTHÄLT
DEVICE FOR INTRODUCING AN IUD, AND MEDICAL KIT INCLUDING SUCH DEVICE

(30) Priorité: 18.10.2012 FR 1259949
(43) Date de publication de la demande: 23.04.2014
(73) Titulaire: CL Investissements, 03700 Serbannes (FR)
(72) Inventeur: Lafont, Charles-Dominique, 03700 Serbanne (FR)
(74) Mandataire: Lavoix

(56) Documents cités:
- FR-A1- 2 806 296
- US-A- 3 918 444

## Description

La présente invention concerne un dispositif de pose d'un stérilet dans l'utérus d'une femme, ainsi qu'un kit médical comportant un tel dispositif.

L'invention s'intéresse plus spécifiquement aux stérilets qui, au repos, présentent une configuration déployée en forme globale de T. Ces stérilets comprennent une tige à une extrémité de laquelle s'étendent transversalement deux bras flexibles, à même d'être pliés contre la tige de manière élastique. Par effet ressort, les bras assurent le maintien en position du stérilet dans l'utérus d'une femme, du fait de leur flexibilité.

De manière connue en soi, ces stérilets permettent d'induire un effet contraceptif. En effet, la présence du stérilet dans l'utérus modifie mécaniquement la configuration de l'utérus. Et, en intégrant typiquement du cuivre au stérilet, un effet biochimique altère la fonction et la viabilité des spermatozoïdes.

La pose de ce type de stérilet est effectuée par un médecin, classiquement à l'aide d'un dispositif dédié, à usage unique, dans lequel le stérilet est généralement préchargé dans sa configuration déployée. Ce dispositif comporte ainsi un inserteur rigide, dans un alésage traversant duquel est agencé le stérilet à poser. Du côté distal, l'alésage de l'inserteur débouche dans une canule souple, qui est solidaire de l'inserteur et qui, lors de la pose, est introduite dans l'utérus, à travers le canal cervical. Du côté proximal, l'alésage reçoit un poussoir qui est déplaçable en translation vers le côté distal de manière à pouvoir atteindre le stérilet et le pousser progressivement hors de l'alésage puis hors de la canule. Au moment où le poussoir commence d'agir sur le stérilet, plus précisément sur l'extrémité de sa tige, opposée aux bras flexibles, le médecin doit appliquer un effort de poussée relative entre le poussoir et l'inserteur, qui est important car l'entraînement translatif de la tige du stérilet s'accompagne du pliage des bras contre cette tige, par butée de ces bras contre des parois dédiées de l'inserteur. La contrainte en compression, subie alors par la tige du stérilet entre ses deux extrémités, est telle qu'un flambage de la tige se produit, avec le risque que l'extrémité de la tige, opposée aux bras, se décale transversalement vis-à-vis de l'extrémité distale du poussoir, induisant alors une mauvaise transmission d'effort entre eux ou, plus généralement, un comportement dégradé du dispositif, éloigné du comportement de fonctionnement normal. Pour le médecin, cette situation l'incite à augmenter l'effort d'entraînement qu'il applique entre le poussoir et l'inserteur, avec le risque réel de perforer une paroi de l'utérus ou le canal cervical: c'est typiquement le cas lorsque, après avoir ainsi dû « forcer » pour que les bras se replient et que le stérilet commence de s'engager dans la canule souple, le médecin continue d'appliquer un effort de poussée élevé en vue de vaincre une résistance à l'avancement du stérilet, cette résistance n'étant plus due à la nécessité de replier les bras du stérilet, mais étant due à un coudage de la canule contre la paroi du canal cervical de par la configuration géométrique curviligne de ce dernier.

Dans ce contexte, US-A-3 918 444, qui divulgue un dispositif de pose tel que défini au préambule de la revendication 1 annexée, propose une alternative qui consiste à entraîner la tige du stérilet aux fins du pliage des bras du stérilet, non pas en poussant cette tige par appui axial contre l'extrémité de cette dernière, opposée aux bras, mais en poussant la tige directement au niveau de son extrémité d'où s'étendent les bras. Pour ce faire, le poussoir du dispositif de pose de US-A-3 918 444 est prévu creux sur toute sa longueur, de manière à y loger la totalité de la tige du stérilet, si bien que seuls les bras du stérilet émergent à l'extérieur du poussoir creux et que la transmission d'effort entre le poussoir et le stérilet est réalisée au niveau de la zone de jonction entre la tige et chaque bras, chacune de ces deux zones de jonction étant d'ailleurs reçue dans une encoche correspondante, prévue à l'extrémité distale du poussoir creux. Les deux zones de jonction sont donc soumises à des contraintes localisées conséquentes, avec des risques évidents de rupture des bras. De plus, cette solution conduit nécessairement à un diamètre élevé pour l'inserteur du dispositif car l'alésage de cet inserteur doit être dimensionné en diamètre pour laisser passer conjointement la tige du stérilet, les bras repliés sur cette tige et le poussoir creux interposé radialement entre la tige et chaque bras.

De son côté, FR-A-2 806 296 s'intéresse à la pose d'un autre type de stérilet en T, à savoir les stérilets qui, lors de leur pose, ont leurs bras qui ne sont pas repliés contre leur tige comme décrit jusqu'ici, mais qui sont amenés l'un contre l'autre, dans le prolongement distal de leur tige. En pratique, pour amener le stérilet dans une configuration où les bras sont agencés dans le prolongement distal de la tige, les bras sont, moyennant la traction du stérilet en direction proximale, rétractés à l'intérieur d'un tube qui est prévu suffisamment souple pour être introduit dans l'utérus. La libération du stérilet dans l'utérus est réalisée en le repoussant en direction distale, ce qui fait émerger les bras depuis l'extrémité distale du tube et leur permet de reprendre élastiquement leur configuration en T, sans avoir besoin de transmettre à la tige un effort de poussée important puisque le stérilet est entraîné jusqu'à l'extérieur du tube sans rencontrer d'obstacle. Cependant, la précision d'implantation obtenue est souvent mauvaise, notamment du fait du faible rappel élastique des bras depuis leur configuration dans le prolongement distal de la tige.

Le but de la présente invention est d'améliorer les dispositifs de pose de stérilets dont les bras sont à replier contre la tige des stérilets lors de la pose, en sécurisant et facilitant leur manipulation par les médecins, notamment pour éviter toute perforation.

A cet effet, l'invention a pour objet un dispositif de pose d'un stérilet dans l'utérus d'une femme, ce stérilet présentant au repos une forme globale de T et comprenant un tige à une extrémité de laquelle s'étendent transversalement deux bras à même d'être pliés de manière élastique contre la tige, lequel dispositif étant tel que défini à la revendication 1.

L'invention a également pour objet un kit médical, tel que défini à la revendication 8.

Une des idées à la base de l'invention est de chercher à fiabiliser la transmission d'effort entre la partie terminale distale du poussoir et la partie proximale de la tige d'un stérilet à poser. L'invention prévoit à cet effet de réaliser, en creux dans le chant d'extrémité libre de la partie terminale distale du poussoir, une cavité allongée dans la direction proximo-distale, qui est fermée du côté proximal par un fond plat, et dans laquelle une partie terminale de la tige, opposée aux bras et d'une longueur substantielle, se trouve logée de façon sensiblement complémentaire. De cette façon, lorsqu'un effort important de poussée relative entre le poussoir et l'inserteur est appliqué par le médecin, typiquement pour contraindre les bras du stérilet à se replier en vue de faire passer le stérilet dans la partie terminale distale de l'alésage de l'inserteur, toute la partie terminale précitée de la tige du stérilet est, en quelque sorte, enveloppée fermement ou gainée par la paroi de cette cavité, tandis que l'appui axial du fond de cette cavité contre l'extrémité, opposée aux bras, de la tige du stérilet assure une bonne transmission d'effort à la tige, notamment en évitant son décalage transversal par rapport à la partie terminale distale du poussoir et en limitant de ce fait le flambage de la tige. L'effort que doit appliquer le médecin au dispositif reste donc raisonnable, en limitant le risque d'une mauvaise manipulation conduisant à une perforation de l'utérus ou du canal cervical.

Des caractéristiques additionnelles avantageuses du dispositif et du kit conformes à l'invention sont spécifiées aux revendications 2 à 7, 9 et 10.

L'invention sera mieux comprise à la lecture de la description qui va suivre, donnée uniquement à titre d'exemple et faite en se référant aux dessins sur lesquels :
- la figure 1 est une vue éclatée en perspective d'une partie d'un dispositif conforme à l'invention, associé à un stérilet ;
- la figure 2 est une coupe selon le plan II de la figure 1 ;
- les figures 3 et 4 sont des coupes selon respectivement les lignes III-III et IV-IV de la figure 2 ;
- la figure 5 est une vue schématique du dispositif complet dont une partie est visible à la figure 1, cette vue schématique combinant, d'une part, une élévation, selon la flèche V indiquée à la figure 1, du stérilet ainsi que d'un poussoir appartenant au dispositif, et, d'autre part, une coupe, selon le plan V' indiqué à la figure 1, du reste de ce dispositif ; et
- la figure 6 est une vue analogue à la figure 5, montrant le dispositif dans une configuration d'utilisation différente de celle montrée à la figure 5.

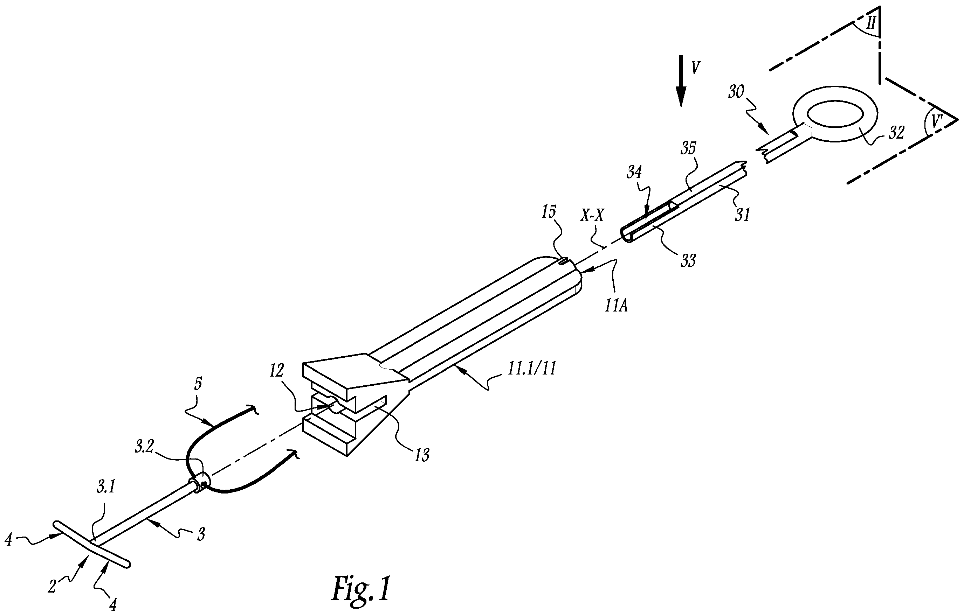

Sur les figures 1 à 6 est représenté un dispositif 1 permettant de poser un stérilet 2 dans l'utérus d'une femme.

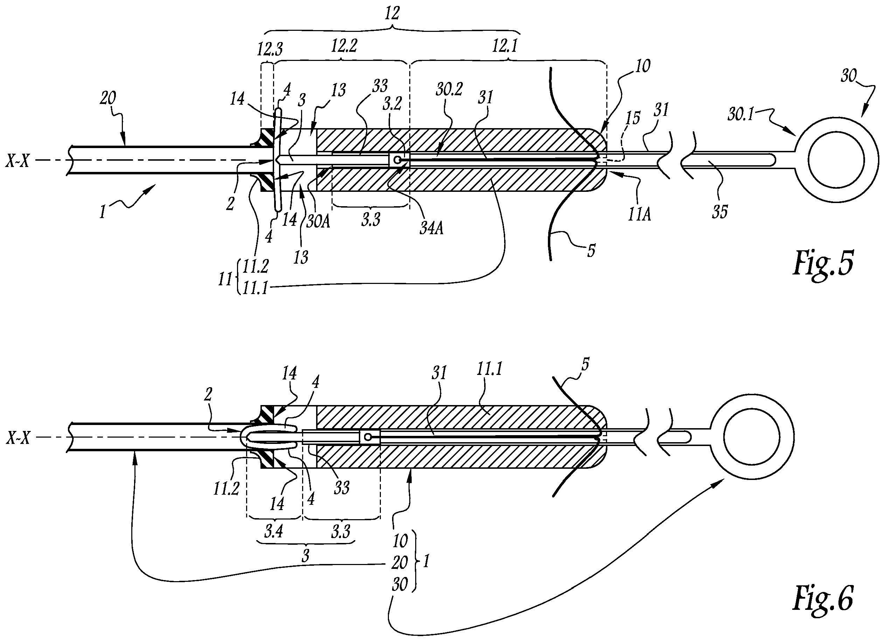

Le stérilet 2, qui est connu en soi, comporte une tige 3 présentant deux extrémités longitudinales opposées 3.1 et 3.2. A son extrémité 3.1, la tige 1 est pourvue de deux bras opposés 4, qui sont respectivement situés de part et d'autre de la tige 3 et qui s'étendent chacun depuis l'extrémité 3.1, transversalement à la tige. Ces bras 4 sont flexibles, dans le sens où, par déformation élastique, notamment de la zone de liaison entre l'extrémité 3.1 de la tige 3 et chacun de ces bras, chaque bras est conçu pour être plié contre la tige, en étant rabattu le long de cette tige. Au repos, c'est-à-dire en l'absence de toute contrainte de déformation appliquée au stérilet 1, en particulier aux bras 4, ces bras sont déployés vis-à-vis de la tige 3 de sorte que la tige et les bras présentent une forme conjointe globalement en T, comme représenté sur les figures 1 et 5, étant remarqué que, à l'inverse, la figure 6 montre une configuration déformée du stérilet 1, comme expliqué plus en détail par la suite.

Par ailleurs, également de manière bien connue, l'extrémité 3.2 de la tige 3 est pourvue d'un fil 5 utilisé pour retirer le stérilet 1 depuis une cavité utérine à l'intérieur de laquelle ce stérilet a été préalablement posé.

A titre d'exemple, la tige 3 et les bras 4 sont réalisés d'une seule pièce en polyéthylène, le fil 5 étant un fil de nylon (marque déposée).

On notera que les spécificités du stérilet 2 ne sont pas limitatives de la présente invention. Autrement dit, l'invention n'est pas limitée aux stérilets identiques à celui montré sur les figures, de très nombreuses variantes de réalisation étant envisageables. D'ailleurs, le stérilet 2 montré sur les figures est typiquement pourvu d'éléments en cuivre, rapportés autour de la tige 3 et/ou autour des bras 4, qui ne sont pas montrés sur les figures.

Comme bien visible sur les figures 1 et 5, le dispositif 1 comporte trois composants principaux, à savoir un inserteur 10, une canule 20 et un poussoir 30, qui vont être détaillés ci-après. Globalement, l'inserteur 10 peut être qualifié de rigide tandis que la canule 20 peut être qualifiée de souple, dans le sens où, en service, l'inserteur 10 n'est pas substantiellement déformé, tandis que la canule 20 est prévue pour être déformée de manière souple pour le passage du canal cervical. Le piston 30, qui, comme expliqué plus en détail par la suite, est destiné à être engagé à travers l'inserteur 10 et à travers la canule 20, peut être qualifié de semi-rigide, par comparaison à l'inserteur et à la canule.

Comme représenté sur les figures 1 et 5, l'inserteur 10 comporte un corps rigide 11 qui, dans l'exemple de réalisation considéré ici, est constitué d'une partie principale 11.1, qui est la seule partie du corps 11 montrée sur la figure 1, et une partie 11.2 de connexion mécanique avec la canule 20. En service, c'est-à-dire lorsque le dispositif 1 est mis à la disposition d'un médecin en vue de poser le stérilet 2, le corps 11 est rigidement d'un seul tenant, ce qui revient à dire que ses parties 11.1 et 11.2 sont solidarisées fixement et à demeure l'une à l'autre. En pratique, diverses possibilités d'assemblage des parties 11.1 et 11.2 l'une à l'autre sont envisageables, sans être limitatives de la présente invention.

Le corps 11 délimite un alésage traversant 12 centré sur un axe proximo-distal X-X. De manière classique, le terme « proximal » désigne une direction orientée vers le médecin, tandis que le terme « distal » désigne une direction de sens opposé, autrement dit orientée vers l'utérus d'une femme traitée. L'alésage 12 traverse ainsi de part en part le corps 11, plus précisément en traversant successivement les parties 11.1 et 11.2 du corps 11. Comme représenté sur la figure 5, l'alésage 12 inclut, successivement suivant son axe X-X, une partie terminale proximale 12.1, une partie intermédiaire 12.2 et une partie terminale distale 12.3. Dans l'exemple de réalisation considéré ici, la partie terminale proximale 12.1 de l'alésage 12 est délimitée exclusivement par la partie 11.1 du corps 11, tandis que la partie terminale distale 12.3 est délimitée exclusivement par la partie de corps 11.2 Et la partie intermédiaire 12.2 de l'alésage 12 est délimitée conjointement par les parties 11.1 et 11.2 du corps 11.

Les parties 11.1 et 11.2 du corps 11 délimitent également conjointement deux lumières 13, qui sont chacune agencées transversalement à l'alésage 12, en étant diamétralement opposées l'une à l'autre par rapport à l'axe X-X, et qui débouchent chacune dans la partie intermédiaire 12.2 de l'alésage 12. Comme bien visible sur les figures 1 et 5, chacune des lumières 13 relie ainsi la partie intermédiaire 12.2 de l'alésage 12 à l'extérieur du corps 11 : en service, dans la configuration du dispositif 1 montrée à la figure 5, le stérilet 2 est agencé au niveau de la partie intermédiaire 12.2 de l'alésage 12, de manière que, d'une part, la tige 3 du stérilet 2 est reçue de manière sensiblement coaxiale dans la partie intermédiaire 12.2 de l'alésage 12, en s'étendant sur toute la dimension axiale de cette partie intermédiaire, et, d'autre part, les bras 4 du stérilet 2 sont respectivement reçus dans les lumières 13.

Une paroi distale 14 de chaque lumière 13, qui est portée par la partie 11.2 du corps 11, forme une butée, suivant la direction de l'axe X-X et vers le côté distal, pour le bras correspondant 4, de sorte que, lorsque le stérilet 2 est déplacé depuis la partie intermédiaire 12.2 de l'alésage 12 vers la partie distale 12.3 de cet alésage, autrement dit lorsque le stérilet 2 est déplacé vis-à-vis de l'inserteur 10 depuis sa position de la figure 5 vers sa position de la figure 6, les parois distales respectives 14 des lumières 13 plient respectivement les bras 4 contre la tige 3, par déformation élastique de ces bras.

Comme bien visible à la figure 1 et comme montré uniquement en pointillés à la figure 5, la paroi de la partie de corps 11.1, délimitant la partie terminale proximale 12.1 de l'alésage 12, est pourvue d'une encoche traversante 15. Ainsi, la partie terminale proximale 12.1 de l'alésage 12 débouche sur l'extérieur du corps 11 à la fois suivant la direction de l'axe X-X, dans le prolongement axial de l'alésage vers le côté proximal, et transversalement à cet axe, via l'encoche précitée 15. Cette encoche 15 est située à l'extrémité proximale du corps 11, dans le sens où cette encoche débouche sur le chant d'extrémité proximale 11A du corps 11 de l'inserteur 10. De la sorte, en service, l'encoche 15 est à même de recevoir le fil 5, comme montré sur la figure 5, le fil étant introduit à l'intérieur de l'encoche depuis le chant d'extrémité proximale 11A de l'inserteur 10, en s'étendant de part et d'autre de cette encoche, c'est-à-dire en s'étendant à la fois à l'intérieur de l'alésage 12 et à l'extérieur du corps 11.

Pour des raisons qui apparaitront plus loin, liées à la manipulation du dispositif 1, l'encoche 15 occupe avantageusement une position angulaire, autour de l'axe X-X, située à sensiblement 90° des positions angulaires respectives des lumières 13, comme bien visible sur la figure 1.

En pratique, bien que non visible sur les figures, le corps 11 est avantageusement pourvu d'une seconde encoche, fonctionnellement similaire à l'encoche 15 décrite ci-dessus et structurellement identique à cette dernière, les deux encoches étant diamétralement opposées vis-à-vis de l'axe X-X. Bien entendu, lorsque le dispositif 1 est utilisé, une seule de ces deux encoches reçoit le fil 5, l'autre encoche n'étant pas utilisée.

Comme représenté uniquement sur les figures 5 et 6, la canule souple 20 est agencée du côté distal de l'inserteur 10, en s'étendant dans le prolongement axial de l'alésage 12. L'extrémité proximale de la canule 20 est solidarisée à demeure au corps 11, plus précisément à la partie 11.2 de ce corps, de sorte que la partie terminale distale 12.3 de l'alésage 12 débouche, du côté distal, à l'intérieur de la canule. En pratique, diverses formes de connexion entre la canule 20 et l'inserteur 10 sont envisageables, sans être limitative de la présente invention.

De manière connue en soi, la canule 20 est dimensionnée en longueur de manière que cette canule puisse être introduite à l'intérieur de l'utérus d'une femme, en s'étendant au moins du col de l'utérus à la paroi anatomique opposée, autrement dit à proximité des trompes de Fallope. Suivant une disposition avantageuse, non représentée sur les figures, la canule 20 est extérieurement graduée pour que le médecin puisse apprécier la longueur de la partie proximale de cette canule, introduite dans l'utérus d'une patiente au cours de l'utilisation du dispositif 1.

Comme représenté sur les figures 1 à 4, le poussoir 30 comprend principalement une tige pleine 31 qui, en service, s'étend en longueur et de manière centrée sur l'axe X-X.

A son extrémité proximale, la tige 31 porte rigidement une bague 32 qui, conjointement avec l'extrémité proximale de la tige 31, forme une partie terminale proximale 30.1 du poussoir 30. Cette bague 32 est destinée à être saisie par une main du médecin, l'un de ses doigts pouvant être introduit à travers la bague. A titre de variante non représentée, d'autres formes de réalisation, que la bague 32, peuvent être envisagées pour la partie terminale proximale 30.1 du poussoir 30, du moment que cette partie terminale proximale soit avantageusement conformée pour faciliter la manipulation du poussoir 30 par le médecin.

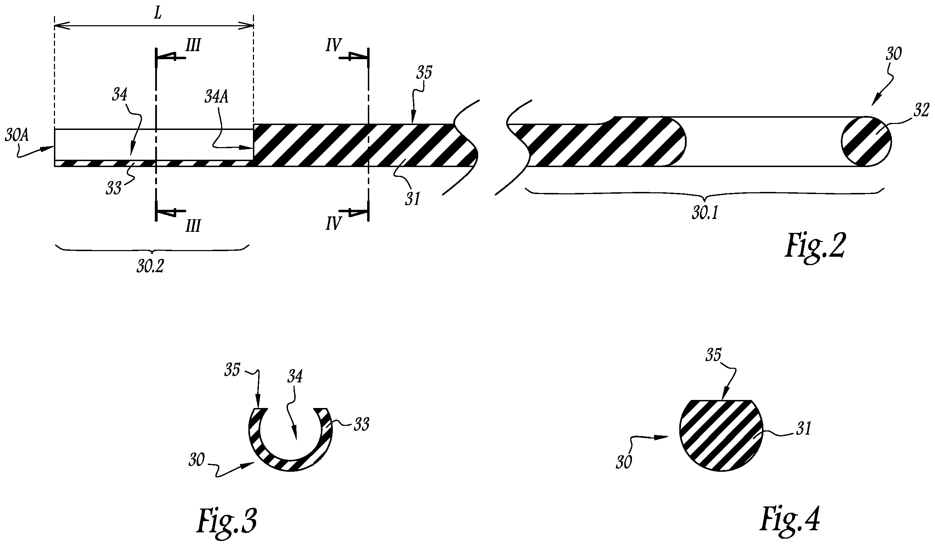

A son extrémité distale, la tige 31 est prolongée, vers le côté distal, par une paroi semi-tubulaire 33, qui est alignée avec la tige 31 et dont la face extérieure s'étend dans le prolongement axial de la face extérieure de la tige 31. Cette paroi semi-tubulaire 33 forme, conjointement avec l'extrémité distale de la tige 31, une partie terminale distale 30.2 du poussoir 30 : ainsi, suivant l'axe X-X, la paroi 33 relie l'un à l'autre le chant d'extrémité libre 30A de cette partie terminale distale 30.2 du poussoir 30 et le chant d'extrémité distale de la tige 31, en présentant entre eux une dimension correspondante notée L sur la figure 2.

La paroi semi-tubulaire 33 délimite intérieurement une cavité 34 qui s'étend sur toute l'étendue axiale de cette paroi, autrement dit avec une dimension longitudinale égale à la dimension précitée L. Du côté distal, cette cavité 34 débouche axialement sur le chant d'extrémité libre 30A du poussoir 30 tandis que, du côté proximal, cette cavité 34 est fermée par un fond plat 34A, qui est constitué par le chant d'extrémité distale de la tige 31 et qui est avantageusement agencé de manière sensiblement perpendiculaire à la direction longitudinale du poussoir 30.

Suivant une disposition optionnelle avantageuse, la tige 31 présente extérieurement un méplat 35 qui s'étend sur sensiblement toute l'étendue axiale de la tige 31, ainsi que sur la partie terminale distale 30.2 du poussoir 30. Au niveau de la cavité 34, le méplat 35 ouvre cette cavité transversalement sur l'extérieur, en interrompant la paroi 33 suivant sa périphérie pour lui donner sa forme semi-tubulaire, comme bien visible que la figure 3.

En dehors du méplat 35, la cavité 34 présente, en coupe transversale au poussoir 30, comme montré sur la figure 3, un profil intérieur qui est complémentaire du profil transversal extérieur de la tige 3 du stérilet 2 : ainsi, comme représenté sur la figure 5, la cavité 34 est dimensionnée pour recevoir de manière sensiblement complémentaire la tige 3 du stérilet 2, plus précisément une partie terminale 3.3 de cette tige, opposée aux bras 4. Dans l'exemple de réalisation considérée sur les figures, les profils transversaux précités sont à base circulaire, mais d'autres formes géométriques sont envisageables. Dans tous les cas, lorsque la partie terminale 3.3 de la tige 3 du stérilet 2 est reçue à l'intérieur de la cavité 34, la paroi 33 de cette dernière gaine, à un jeu fonctionnel près, cette partie terminale 3.3 de la tige 3, en alignant l'une avec l'autre cette tige 3 et la tige 31 du poussoir 30, c'est-à-dire en rendant ces deux tiges coaxiales, tandis que le reste de la tige 3, qui est référencé 3.4 et qui inclut l'extrémité de tige 3.1 portant les bras 4, s'étend à l'extérieur de la cavité 34.

Par ailleurs, également en dehors du méplat 35, la paroi 33 présente, en coupe transversale au poussoir 30, comme montré sur les figures 3 et 4, un profil extérieur qui est complémentaire du profil transversal intérieur de l'alésage 12, tout du moins du profil transversal intérieur commun des parties terminale proximale 12.1 et intermédiaire 12.2 de cet alésage 12. Ainsi, on comprend que cette paroi 33 est conçue pour être reçue de manière sensiblement complémentaire et coaxiale dans les parties précitées 12.1 et 12.2 de l'alésage 12, comme représenté sur la figure 5. Dans l'exemple de réalisation considéré ici, les profils transversaux précités sont à base circulaire, mais, comme précédemment, d'autres formes géométriques sont envisageables.

Un exemple d'utilisation du dispositif 1, visant à poser le stérilet 2 dans l'utérus d'une femme, va maintenant être décrit.

Initialement, on considère que le dispositif 1 et le stérilet à poser 2 sont tels que montrés à la figure 5. En pratique, le dispositif 1 et le stérilet 2 sont mis à disposition du médecin sous forme d'un kit médical, dans un emballage stérile que le médecin va ouvrir au début de l'intervention de pose. En particulier, les parties 11.1 et 11.2 du corps 11 sont préassemblées, avec le stérilet 2 préchargé dans l'inserteur 10. Le cas échéant, pour que le dispositif 1 et le stérilet 2 soient dans la configuration de la figure 5, le médecin introduit le poussoir 30 dans l'alésage 12, en engageant la partie terminale distale 30.2 de ce poussoir dans la partie terminale proximale 12.1 de l'alésage 12, jusqu'à mettre en butée axiale le fond 34A de la cavité 34 contre l'extrémité 3.2 de la tige 3. Aussi, dans cette configuration de la figure 5, on remarque que le stérilet 2 est dans un état déployé, avec ses bras 4 reçus dans les lumières 13 de l'inserteur 10, tandis que sa tige 3 est reçue dans la partie intermédiaire 12.2 de l'alésage 12, la partie terminale 3.3 de cette tige, opposée aux bras 4, étant reçue dans la cavité 34, la paroi 33 se trouvant ainsi radialement interposée entre cette partie terminale de la tige 3 et la paroi de la partie intermédiaire 12.2 de l'alésage 12.

Au cours de l'intervention de pose, le médecin applique au dispositif 1 un effort de poussée relative entre l'inserteur 10 et le poussoir 30, orienté suivant l'axe X-X et tourné du côté distal : autrement dit, le médecin entraine en translation relative, le long de l'axe X-X, l'inserteur 10 et le poussoir 30, de manière à enfoncer davantage le piston dans l'inserteur. Comme visible par comparaison des figures 5 et 6, cet effort de poussée, appliqué au dispositif 1 par le médecin, vise à faire passer le stérilet 2 de la partie intermédiaire 12.2 de l'alésage 12 jusqu'à l'intérieur de la canule 20, via la partie distale 12.3 de l'alésage 12. Comme expliqué plus haut, ce déplacement du stérilet 2 est accompagné du pliage des bras 4 contre la tige 3, sous l'action des parois distales respectives 14 des lumières 13. Pour ce faire, l'effort de poussée est transmis à la tige 3 du stérilet 2, par appui axial du fond 34A de la cavité 34 contre l'extrémité 3.2 de la tige 3. Grâce à l'alignement de la tige 3 sur ce fond 34A, la transmission d'effort est efficace, tout en limitant les risques de flambage de la tige 3, d'autant que la partie terminale de cette dernière reste gainée par la paroi 33 du poussoir 30 pendant son entraînement par rapport à l'inserteur.

On comprend que le positionnement, centré sur l'axe X-X, de la tige 3 du stérilet 2 est favorisé par la dimension longitudinale L de la cavité 34. Cet effet est notamment significatif des lors que cette dimension L est strictement supérieure au diamètre extérieur, en dehors du méplat 35, de la tige 31 du poussoir 30, plus généralement strictement supérieure à la dimension transversale maximale de la partie terminale distale 30.2 du poussoir 30. Préférentiellement, cet effet est décuplé en dimensionnant en longueur la cavité 34 en tenant compte de la dimension longitudinale de la tige 3 du stérilet 2 : ainsi, suivant une forme de réalisation préférée, la dimension longitudinale L est supérieure à 25 %, voire 50 % de la dimension longitudinale de la tige 3, mesurée entre ses extrémités 3.1 et 3.2. Dans tous les cas, pour éviter tout coincement du stérilet avec le poussoir, l'étendue longitudinale de la partie terminale 3.4 de la tige, c'est-à-dire de la partie de tige non logée à l'intérieur de la cavité 34, est strictement supérieure à la longueur de chaque bras 4, de façon que ces bras peuvent se plier contre la tige 3 sans interférer avec la paroi 33 du poussoir 30. Cela revient à dire que la somme de la dimension L et de la longueur des bras 4 est strictement inférieure à la dimension longitudinale de la tige 3, étant entendu que les bras 4 présentent chacun la même longueur qui est mesurée entre l'extrémité de tige 3.1 et l'extrémité libre de l'un ou l'autre des bras.

Au cours de l'intervention de pose, en particulier durant l'application de l'effort de poussée qui vient d'être décrit, le fil 5 du stérilet 2 court à l'intérieur des parties intermédiaire 12.2 et proximale 12.1 de l'alésage 12, sur le méplat 35, en émergeant de cet alésage via l'encoche 15. Grâce à cet encoche, le médecin peut ainsi contrôler facilement le positionnement angulaire, autour de l'axe X-X, du fil 5 pendant le déplacement relatif entre l'inserteur 10 et le poussoir 30, en particulier lors de l'introduction de la partie terminale distale 30.2 du poussoir 30 dans l'alésage 12 pour atteindre le stérilet 2. Les risques de pincer, voire de coincer le fil 5 entre le poussoir et la paroi de l'alésage sont ainsi évités.

Bien entendu, divers aménagements et variantes au dispositif 1 décrit jusqu'ici sont par ailleurs envisageables.

## Revendications

1. Dispositif (1) de pose d'un stérilet (2) dans l'utérus d'une femme, ce stérilet présentant au repos une forme globale de T et comprenant un tige (3) à une extrémité (3.1) de laquelle s'étendent transversalement deux bras (4) à même d'être pliés de manière élastique contre la tige,
lequel dispositif comporte :
- un inserteur rigide (10), qui délimite, d'une part, un alésage traversant (12), centré sur un axe proximo-distal (X-X) et incluant, suivant cet axe, à la fois une partie intermédiaire (12.2) de réception sensiblement coaxiale de la tige (3) du stérilet à poser (2) et une partie terminale distale (12.3) de passage de la tige et des bras (4) pliés contre cette tige lors de la pose du stérilet, et, d'autre part, deux lumières (13) transversales à l'alésage et débouchant dans la partie intermédiaire de cet alésage, ces lumières étant adaptées à la fois pour recevoir chacune l'un des bras du stérilet à poser lorsque la tige de ce stérilet est dans la partie intermédiaire de l'alésage, et pour plier ces bras contre la tige lorsque la tige est déplacée de la partie intermédiaire à la partie terminale distale de l'alésage, et
- un poussoir (30) d'entrainement du stérilet (2) par rapport à l'inserteur (10), ce poussoir incluant une partie terminale distale (30.2) adaptée pour être déplacée dans l'alésage (12) de manière à pousser la tige (3) du stérilet à poser (2), depuis la partie intermédiaire (12.2) de l'alésage hors de l'inserteur via la partie terminale distale (12.3) de l'alésage, en contraignant les bras (4) du stérilet à se plier contre la tige sous l'action des lumières (13),
la partie terminale distale (30.2) du poussoir (30) délimitant une cavité (34) allongée suivant la direction longitudinale du poussoir, qui débouche sur le chant d'extrémité libre (30A) de la partie terminale (30.2) du poussoir (30), et qui est adaptée pour recevoir de manière sensiblement complémentaire et coaxiale une partie terminale (3.3), opposée aux bras (4), de la tige (3) du stérilet à poser (2),
**caractérisé en ce que** ladite cavité (34) présente, à l'opposé dudit chant d'extrémité libre de la partie terminale du poussoir, un fond plat (34A) d'appui axial contre l'extrémité (3.2), opposée aux bras, de ladite tige.

2. Dispositif suivant la revendication 1, **caractérisé en ce que** la cavité (34) présente une dimension longitudinale (L) strictement supérieure à la dimension transversale maximale de la partie terminale distale (30.2) du poussoir (30).

3. Dispositif suivant l'une des revendications 1 ou 2, **caractérisé en ce que** le fond plat (34A) de la cavité (34) est agencé de manière sensiblement perpendiculaire à la direction longitudinale du poussoir (30).

4. Dispositif suivant l'une quelconque des revendications précédentes, **caractérisé en ce que** le poussoir (30) est extérieurement pourvu d'un méplat (35) qui, au niveau de la cavité (34), ouvre transversalement cette cavité.

5. Dispositif suivant l'une quelconque des revendications précédentes, **caractérisé en ce que** l'alésage (12) inclut en outre une partie terminale proximale (12.1), par laquelle la partie terminale distale (30.2) du poussoir (30) est passée depuis l'extérieur de l'inserteur (10) pour atteindre le reste de l'alésage et pouvoir agir sur la tige (3) du stérilet à poser (2),
et **en ce que** la paroi de l'inserteur (10), qui délimite la partie terminale proximale (12.1) de l'alésage (12), est pourvue d'au moins une encoche traversante (15), qui ouvre transversalement la partie terminale proximale de l'alésage et qui débouche sur le chant d'extrémité proximale (11A) de l'inserteur.

6. Dispositif suivant la revendication 5, **caractérisé en ce que** la ou chaque encoche (15) occupe une position angulaire, autour de l'axe (X-X), située à sensiblement 90° des positions angulaires respectives des lumières (13).

7. Dispositif suivant l'une quelconque des revendications précédentes, **caractérisé en ce que** le dispositif (1) comporte en outre une canule souple (20) de libération du stérilet (2) dans l'utérus, qui est fixée à l'inserteur (10) de manière que la partie terminale distale (12.3) de l'alésage (12) débouche axialement dans cette canule (20).

8. Kit médical, comportant :
- un stérilet (2) qui présente au repos une forme globale de T et qui comprend une tige (3) à une extrémité (3.1) de laquelle s'étendent transversalement deux bras (4) à même d'être pliés de manière élastique contre la tige, et
- un dispositif (1) de pose du stérilet (2) dans l'utérus d'une femme, ce dispositif étant conforme à l'une quelconque des revendications précédentes.

9. Kit suivant la revendication 8, **caractérisé en ce que** la cavité (34) de la partie terminale distale (30.2) du poussoir (30) présente une dimension longitudinale (L) supérieure à 25 %, voire 50%, de la dimension longitudinale de la tige (3) du stérilet (2).

10. Kit suivant la revendication 9, **caractérisé en ce que** la somme de la dimension longitudinale (L) de la cavité (34) et de la longueur des bras (4) du stérilet (2) est strictement inférieure à la dimension longitudinale de la tige (3) du stérilet.

## Patentansprüche

1. Vorrichtung (1) zum Anordnen einer Spirale (2) in dem Uterus einer Frau, wobei diese Spirale im Ruhezustand im Ganzen gesehen eine T-Form aufweist und einen Stab (3) umfasst, an dessen einem Ende (3.1) sich quer zwei Arme (4) erstrecken, die direkt in elastischer Weise gegen den Stab gefaltet werden,
wobei die Vorrichtung umfasst:
- ein starres Einführelement (10), das einerseits eine Querbohrung (12), die auf eine proximodistale Achse (X-X) zentriert ist und dieser Achse folgend sowohl ein Zwischenteil (12.2) zur im Wesentlichen koaxialen Aufnahme des Stabes (3) der anzuordnenden Spirale (2) und ein distales Endteil (12.3) für den Durchgang des Stabes und der gegen diesen Stab gefalteten Arme (4) bei der Anordnung der Spirale einschließt, und andererseits zwei Öffnungen (13) quer zur Bohrung, die in das Zwischenteil dieser Bohrung münden, begrenzt, wobei diese Öffnungen angepasst sind, sowohl jeweils einen der Arme der anzuordnenden Spirale, wenn der Stab dieser Spirale in dem Zwischenteil der Bohrung ist, aufzunehmen als auch diese Arme gegen den Stab zu falten, wenn der Stab von dem Zwischenteil in den distalen Endteil der Bohrung verschoben wird, und
- ein Drückelement (30) für das Mitführen der Spirale (2) in Bezug auf das Einführelement (10), wobei dieses Drückelement ein distales Endteil (30.2) einschließt, das angepasst ist, in der Bohrung (12) verschoben zu werden, um den Stab (3) der anzuordnenden Spirale (2) von dem Zwischenteil (12.2) der Bohrung aus dem Einführelement über das distale Endteil (12.3) der Bohrung zu drücken, wobei die Arme (4) der Spirale gezwungen werden, sich gegen den Stab unter der Wirkung der Öffnungen (13) zu falten,
wobei das distale Endteil (30.2) des Drückelements (30) einen in Richtung der Längsachse des Drückelements langgestreckten Hohlraum (34) begrenzt, der an dem freien Endrand (30A) des Endteils (30.2) des Drückelements (30) mündet und der angepasst ist, im Wesentlichen komplementär und koaxial ein den Armen (4) entgegengesetztes Endteil (3.3) des Stabes (3) der anzuordnenden Spirale (2) aufzunehmen,
**dadurch gekennzeichnet, dass** der Hohlraum (34) entgegengesetzt zu dem freien Endrand des Endteils des Drückelements einen ebenen Boden (34A) der axialen Abstützung gegen das den Armen entgegengesetzte Ende (3.2) des Stabes aufweist.

2. Vorrichtung nach Anspruch 1, **dadurch gekennzeichnet, dass** der Hohlraum (34) eine Längsabmessung (L) aufweist, die streng größer als die maximale Querabmessung des distalen Endteils (30.2) des Drückelements (30) ist.

3. Vorrichtung nach einem der Ansprüche 1 oder 2, **dadurch gekennzeichnet, dass** der ebene Boden (34A) des Hohlraums (34) im Wesentlichen senkrecht zur Längsrichtung des Drückelements (30) angeordnet ist.

4. Vorrichtung nach einem beliebigen der vorhergehenden Ansprüche, **dadurch gekennzeichnet, dass** das Drückelement (30) außen mit einer Abflachung (35) versehen ist, die an dem Hohlraum (34) quer diesen Hohlraum öffnet.

5. Vorrichtung nach einem beliebigen der vorhergehenden Ansprüche, **dadurch gekennzeichnet, dass** die Bohrung (12) außerdem ein proximales Endteil (12.1) einschließt, über das das distale Endteil (30.2) des Drückelements (30) von außerhalb des Einführelements (10) hindurchgeht, um den Rest der Bohrung zu erreichen und auf den Stab (3) der anzuordnenden Spirale (2) einwirken zu können,
und dass die Wand des Einführelements (10), die das proximale Endteil (12.1) der Bohrung (12) begrenzt, mit mindestens einer durchgehenden Aussparung (15) versehen ist, die quer das proximale Endteil der Bohrung öffnet und die an dem proximalen Endrand (11A) des Einführelements mündet.

6. Vorrichtung nach Anspruch 5, **dadurch gekennzeichnet, dass** die oder jede Aussparung (15) eine Winkelposition um die Achse (X-X) herum einnimmt, die im Wesentlichen um 90° versetzt zu den entsprechenden Winkelpositionen der Öffnungen (13) gelegen ist.

7. Vorrichtung nach einem beliebigen der vorhergehenden Ansprüche, **dadurch gekennzeichnet, dass** die Vorrichtung (1) außerdem eine weiche Kanüle (20) zur Freisetzung der Spirale (2) im Uterus aufweist, die an dem Einführelement (10) derart befestigt ist, dass das distale Endteil (12.3) der Bohrung (12) axial in diese Kanüle (20) mündet.

8. Medizinkit, umfassend:
- eine Spirale (2), die im Ruhezustand im Wesentlichen eine T-Form aufweist und die einen Stab (3) umfasst, an dessen einem Ende (3.1) sich quer zwei Arme (4) erstrecken, die direkt gegen den Stab in elastischer Weise faltbar sind, und
- eine Vorrichtung (1) zum Anordnen der Spirale (2) in dem Uterus einer Frau, wobei diese Vorrichtung nach einem beliebigen der vorhergehenden Ansprüche ausgebildet ist.

9. Kit nach Anspruch 8, **dadurch gekennzeichnet**, das der Hohlraum (34) des distalen Endteils (30.2) des Drückelements (30) eine Längsabmessung (L) größer als 25 %, sogar 50%, der Längsabmessung des Stabes (3) der Spirale aufweist.

10. Kit nach Anspruch 9, **dadurch gekennzeichnet, dass** die Summe der Längsabmessung (L) des Hohlraums (34) und der Länge des Armes (4) der Spirale (2) streng kleiner als die Längsabmessung des Stabes (3) der Spirale ist.

## Claims

1. Device (1) for fitting an intrauterine device (2) in the uterus of a woman, the intrauterine device being generally T-shaped at rest and comprising a stem (3) at one end (3.1) of which there extend transversely two arms (4) capable of being folded resiliently against the stem,
which device comprises:
- a rigid inserter (10) which delimits, on the one hand, a through-bore (12) which is centred on a proximodistal axis (X-X) and includes, along that axis, both an intermediate portion (12.2) for substantially coaxially receiving the stem (3) of the intrauterine device to be fitted (2) and a distal terminal portion (12.3) for passage of the stem and of the arms (4) folded against the stem during fitting of the intrauterine device, and, on the other hand, two openings (13) which are transverse to the bore and open in the intermediate portion of the bore, the openings being adapted each to receive one of the arms of the intrauterine device to be fitted when the stem of the intrauterine device is in the intermediate portion of the bore, and also to fold the arms against the stem when the stem is displaced from the intermediate portion to the distal terminal portion of the bore, and
- a pusher (30) for moving the intrauterine device (2) relative to the inserter (10), the pusher including a distal terminal portion (30.2) adapted to be displaced in the bore (12) so as to push the stem (3) of the intrauterine device to be fitted (2) from the intermediate portion (12.2) of the bore out of the inserter *via* the distal terminal portion (12.3) of the bore, forcing the arms (4) of the intrauterine device to fold against the stem under the action of the openings (13),
the distal terminal portion (30.2) of the pusher (30) delimiting a cavity (34) which is elongated in the longitudinal direction of the pusher and opens at the free end edge (30A) of the terminal portion (30.2) of the pusher (30), and which is adapted to receive in a substantially complementary and coaxial manner a terminal portion (3.3), opposite the arms (4), of the stem (3) of the intrauterine device to be fitted (2),
**characterised in that** said cavity (34) has, opposite said free end edge of the terminal portion of the pusher, a flat bottom (34A) for axial abutment against the end (3.2), opposite the arms, of said stem.

2. Device according to claim 1, **characterised in that** the cavity (34) has a longitudinal dimension (L) which is strictly greater than the maximum transverse dimension of the distal terminal portion (30.2) of the pusher (30).

3. Device according to either claim 1 or claim 2, **characterised in that** the flat bottom (34A) of the cavity (34) is arranged substantially perpendicularly to the longitudinal direction of the pusher (30).

4. Device according to any one of the preceding claims, **characterised in that** the pusher (30) is provided on the outside with a flat surface (35) which, in the region of the cavity (34), opens the cavity transversely.

5. Device according to any one of the preceding claims, **characterised in that** the bore (12) further includes a proximal terminal portion (12.1) through which the distal terminal portion (30.2) of the pusher (30) is passed from outside the inserter (10) in order to reach the remainder of the bore and be able to act upon the stem (3) of the intrauterine device to be fitted (2),
and **in that** the wall of the inserter (10), which delimits the proximal terminal portion (12.1) of the bore (12), is provided with at least one through-notch (15) which transversely opens the proximal terminal portion of the bore and which opens at the proximal end edge (11A) of the inserter.

6. Device according to claim 5, **characterised in that** the or each notch (15) occupies an angular position, about the axis (X-X), which is situated at substantially 90° to the respective angular positions of the openings (13).

7. Device according to any one of the preceding claims, **characterised in that** the device (1) further comprises a flexible cannula (20) for releasing the intrauterine device (2) in the uterus, which cannula is fixed to the inserter (10) in such a manner that the distal terminal portion (12.3) of the bore (12) opens axially into the cannula (20).

8. Medical kit comprising:
- an intrauterine device (2) which is generally T-shaped at rest and which comprises a stem (3) at one end (3.1) of which there extend transversely two arms (4) capable of being folded resiliently against the stem, and
- a device (1) for fitting the intrauterine device (2) in the uterus of a woman, the device being in accordance with any one of the preceding claims.

9. Kit according to claim 8, **characterised in that** the cavity (34) of the distal terminal portion (30.2) of the pusher (30) has a longitudinal dimension (L) which is greater than 25%, or even 50%, of the longitudinal dimension of the stem (3) of the intrauterine device (2).

10. Kit according to claim 9, **characterised in that** the sum of the longitudinal dimension (L) of the cavity (34) and the length of the arms (4) of the intrauterine device (2) is strictly less than the longitudinal dimension of the stem (3) of the intrauterine device.
